# EUROPÄISCHE PATENTSCHRIFT

(11) **EP 0 395 851 B1**
(45) Veröffentlichungstag und Bekanntmachung des Hinweises auf die Patenterteilung: **26.06.1996**
(21) Anmeldenummer: 90104212.7
(22) Anmeldetag: 05.03.1990
(51) Int. Cl.: C07K 14/415, C07K 1/14, A61K 38/16

(54) **Glykoproteine aus Avena sativa, Verfahren zu ihrer Herstellung und ihre Verwendung als pharmazeutischer Wirkstoff**
Avena sativa glycoproteins, procedure for obtaining them, and their use as medicines
Glycoprotéines d'Avena sativa procédé pour les obtenir et leur utilisation comme médicaments

(30) Priorität: 29.04.1989 DE 3914354
(43) Veröffentlichungstag der Anmeldung: 07.11.1990
(73) Patentinhaber: SCHAPER & BRÜMMER GMBH & CO. KG, D-38259 Salzgitter (DE)
(72) Erfinder: Beuscher, Norbert, Dipl.-Biol.-Dr., D-3320 Salzgitter Bad (DE); Scheit, Karl-Heinz, Dipl.-Chem.-Prof.-Dr., D-3400 Göttingen (DE)
(74) Vertreter: Lins, Edgar, Dipl.-Phys. Dr.jur.

(56) Entgegenhaltungen:
- PLANT PHYSIOL., Band 75, Nr. 1, 1984, Seiten 225-227; K. ADELI et al.: "Characterization of oat vicilin-like polypeptides"

## Beschreibung

Die Erfindung betrifft ein Glykoprotein mit immunomodulierenden Eigenschaften mit einem Molekulargewicht von 38 kD, herstellbar aus einem Extrakt der Früchte von Avena sativa, mit einem Anteil von Aminosäureresten von 58,4 % und von Zuckerresten von 41,6 %, mit einem apparenten Molekulargewicht der Polypeptidkette von 22,2 kD, mit neutralen Hexosen in der Zusammensetzung Glukose 45 %, Galaktose 8 %, Mannose 31 %, Arabinose 15 % und einem Gehalt an Osaminen von unter 0,1 % und mit Aminosäureverhältnissen von Aspartat zu Glutamat von 0,97, Alanin zu Valin von 1,03, Valin zu Histidin von 10,6, Valin zu Lysin von 2,3 und Valin zu Arginin von 3,5. Die Erfindung betrifft ferner ein Verfahren zur Herstellung eines Glycoproteins sowie ein Arzneimittel mit dem Glycoprotein als Wirkstoff und dessen Verwendung zur Herstellung eines immunmodulierenden Arzneimittels.

Das neu aufgefundene Glykoprotein ist wasserlöslich und hat ein Molekulargewicht von 38 kD. Es weist Aminosäureverhältnisse von Aspartat zu Glutamat von 0,97, Alanin zu Valin von 1,03, Valin zu Histidin von 10,6, Valin zu Lysin von 2,3 und Valin zu Arginin von 3,5 auf. Für das apparente Molekulargewicht der Polypeptidkette (nur Aminosäurereste) ergibt sich somit ein Wert von 22,2 kD. Das Molekulargewicht des Glykoproteins wird aus den Ergebnissen der Aminosäure- und der Zuckeranalyse ermittelt. Das erfindungsgemäße Glykoprotein enthält darüber hinaus neutrale Hexosen, und zwar Glukose (45 %), Galaktose (8 %), Mannose (31 %) und Arabinose (15 %). Der Gehalt an Osaminen (Glukosamin und Galaktosamin) liegt unter 0,1 %. Der Anteil der Aminosäurereste beträgt 58,4 % und der Zuckerreste 41,6 %. Die ermittelten Werte sind durch Gaschromatographie und Aminosäureanalyse der Totalhydrolysate gefunden worden.

Das erfindungsgemäße Glykoprotein wird somit von einer Polypeptidkette gebildet, die mit dem Polysaccharidanteil gekoppelt ist.

Bei dem bevorzugten erfindungsgemäßen Glykoprotein besteht die Proteinfraktionen aus etwa 11 Mol-% Asparaginsäure, 11 Mol-% Glutaminsäure und etwa 8 Mol-% Threonin. Glukose, Galaktose, Mannose und Arabinose liegen vorzugsweise etwa im Verhältnis 6 : 1 und 4 : 2 vor.

Das erfindungsgemäße Glykoprotein kann aus den Früchten von Avena sativa extrahiert werden.

In dem hierfür bevorzugten erfindungsgemäßen Verfahren werden die hochmolekularen Anteile des Extraktes zu deren Weiterverarbeitung ausgefiltert, vorzugsweise durch Diafiltration. Anschließend werden die hochmolekularen Anteile mit einem für Glykoproteine geeigneten Fällmittel, vorzugsweise Ammoniumsulfat gefällt. Der entstandene Niederschlag wird beispielsweise durch Zentrifugation gewonnen, in Wasser gelöst und dialysiert, um verunreinigende Salze zu entfernen.

Vorzugsweise wird die gereinigte Lösung zur Aufbewahrung lyophilisiert und anschließend erneut in Lösung gebracht. Daraufhin erfolgt die Trennung der Komponenten nach Molekulargewichtsbereichen und das Separieren des Molekulargewichtsbereichs 38 kD. Diese Trennung erfolgt vorzugsweise über eine Chromatographie an Gelen. Die aufgefundene aktive Fraktion kann dann eingeengt und ggf. erneut lyophilisiert werden.

Es kann vorteilhaft sein, vor der Durchführung des erfindungsgemäßen Verfahrens den Extrakt von hochmolekularen Begleitstoffen wie Lipiden und Nucleinsäuren zu befreien. Hierzu kann eine Fällung mit quaternärem Ammonium, z. B. mit Pyridylcetylamoniumchlorid, vorzugsweise jedoch Trimethylcetylammoniumbromid und ein anschließendes Zentrifugieren zum Absondern der gefällten Begleitstoffe durchgeführt werden.

Aus Stabilitätsgründen werden die Fällungen und Zentrifugationen vorzugsweise in der Kälte (bei etwa +4° C) durchgeführt.

Für die Diafiltration, also die Zurückhaltung von Molekülen mit einem Molekulargewicht gleich oder höher einem gegebenen Molekulargewicht, werden definierte poröse Membranen verwendet. Diese können Hollow-Fiber-Patronen sein, bevorzugt ist jedoch die Verwendung von Spiral-Ultrafiltrationsmodulen. Vorzugsweise werden Membranen der Firma Millipore verwendet, die unter der Bezeichnung PTGC im Handel sind. Die Selektionierung der Moleküle mit einem über einem vorgegebenen Molekulargewicht liegenden Molekulargewicht kann selbstverständlich auch mit anderen Verfahren, wie beispielsweise die Chromatographie an einem hydrophilen, polymerisierten Gel, vorgenommen werden.

Für die Trennung der Komponenten nach Molekulargewichten, werden vorzugsweise hydrophile, polymerisierte Gele mit einem Ausschlußvolumen von > 30.000 Dalton verwendet, die durch modifizierte Dextrane gebildet sind. Geeignet sind unter der Bezeichnung "Sephadex G-50" im Handel befindliche Gele.

Die ersten eluierten Fraktionen werden dabei vereinigt, konzentriert und dialysiert. Anschließend erfolgt eine Einengung zur Trockne, die vorteilhafterweise mittels der Gefriertrocknung durchgeführt wird. Die Lyophilisierungen werden in der bekannten Weise mit Gefriersublimationsanlagen durchgeführt, die in durchschnittlicher Größe und auch als kleine Laboratoriumsgeräte im Markt erhältlich sind.

Nach Auflösung des Lyophilisats wird die Gelfiltration vorgenommen. Vorteilhafterweise ist die zu filtrierende Lösung gepuffert und die Elution wird mit dem gleichen Puffer durchgeführt. Bevorzugt wird ein 0,1 M Ammoniumcarbonat-Puffer verwendet. Die Filtration kann mit Hilfe klassischer Verfahren, insbesondere der UV-Spektrometrie, bei 280 nm verfolgt werden. Das hierfür verwendete Gel ist ebenfalls vorzugsweise ein modifiziertes Dextran, das unter der Bezeichnung "Sephacryl S-200" am Markt ist.

Die erfindungsgemäßen Glykoproteine besitzen überraschende pharmakologische Eigenschaften. Sie sind insbesondere mit bemerkenswerten immunmodulierenden Eigenschaften sowie einer guten Verträglichkeit ausgestattet. Diese Eigenschaften rechtfertigen die Verwendung der anmeldungsgemäßen Glykoproteine als Arneimittel. Diese Arzneimittel finden beispielsweise Anwendung bei der Behandlung und Verhütung von bakteriellen bzw. viralen Infektionen bei Mensch und Tier.

In Frage kommt der Einsatz bei akuten und chronischen Infekten. Beispiele von bakteriellen Infekten sind Bronchitis, Angina, Laryngitis, Otitis und Sinusitis, Beispiele für virale Infekte sind solche, die durch Herpes-Viren, Rhinoviren Influenzaviren, HIV oder Cytomegalie-Viren hervorgerufen werden.

Weiterhin können die erfindungsgemäßen Glykoproteine zur Therapie der Infektanfälligkeit aufgrund einer genetisch bedingten oder einer temporären Abwehrschwäche eingesetzt werden. Die übliche Dosis wird, abhängig von dem zu behandelnden Patienten und der jeweiligen zu therapierenden Erkrankung, variieren. So kann sie z. B. beim Menschen zwischen 10 und 200 mg pro Tag bei oraler Verabreichung, 1 bis 15 mg pro Tag bei rektaler Verabreichung und 2 bis 20 mg pro Tag bei paranteraler Verabreichung betragen.

Die entsprechenden pharmazeutischen Zusammensetzungen können z. B. Feststoffe oder Flüssigkeiten sein und in pharmazeutischen Formen vorliegen, die üblicherweise in der Humanmedizin eingesetzt werden, wie z. B. einfache und dragierte Tabletten, Gelkapseln, Granulate, Lösungen, Sirupe, Suppositorien, injizierbare, lyophilisierte oder nicht-lyophilisierte Präparate, Ovula, Cremes, Lotionen, Tropfen, Augentropfen, Aerosole; sie können nach üblichen Methoden hergestellt werden. Der oder die Wirkstoffe können hierbei üblicherweise in derartigen pharmazeutischen Zusammensetzungen verwendeten Exzipientien einverleibt werden, wie Talk, Gummi arabicum, Lactose, Stärke, Magnesiumstearat, Kakaobutter, wäßrige oder nicht-wäßrige Träger, Fettkörper tierischen oder pflanzlichen Ursprungs, Paraffinderivate, Glykole, verschiedene Netz-, Dispergier- oder Emulgiermittel und Konservierungsstoffe.

Die nachstehenden Beispiele erläutern die Erfindung, ohne sie zu beschränken:

### 1. Tabletten mit Avena-Glykoproteinen

Zur Herstellung von Tabletten mit jeweils 200 mg Einzelgewicht, die 50 mg Glykoproteine enthalten, benötigt man

| | |
|---|---|
| Avena-Glykoproteine | 50 mg |
| mikrokristalline Cellulose | |
| z. B. "Avicel ph 102" | 129 mg |
| Polyvinylpyrrolidon (z. B. "Polyplasdone XL") | 20 mg |
| Magnesiumstearat | 1 mg |

### 2. Kapseln mit Avena-Glykoproteinen zu 300 mg

Für eine Kapsel zu 100 mg benötigt man

| | |
|---|---|
| Avena-Glykoproteine | 50 mg |
| Gelbes Bienenwachs | 10 mg |
| Sojabohnenöl | 10 mg |
| Pflanzenöl | 130 mg |
| Kapselhülle | 100 mg |

### 3. Lösung mit Avena-Glykoproteinen

für 100 ml benötigt man

| | |
|---|---|
| Avena-Glykoproteine | 500 mg |
| Sorbit | 10 g |
| Saccharin Natrium | 0,05 g |
| H₂O bidest. | ad 100 ml |

### 4. Injektionsampullen mit Avena-Glykoproteinen

Zur Herstellung von Injetionsampullen mit jeweils 2 ml Inhalt, die 2 mg Glykoproteinen enthalten, benötigt man

| | |
|---|---|
| Avena-Glykoproteine | 2 mg |
| Physiologische NaCl | ad 2,0 ml |

### 5. Infusionslösung mit Avena-Glykoproteinen

Für 500 ml Infusionslösung benötigt man

| | |
|---|---|
| Avena-Glykoproteine | 20 mg |
| Physiologische NaCl | ad 500 ml |

### 6. Salbe mit Avena-Glykoproteinen

Für 100 g Salbe benötigt man

| | |
|---|---|
| Avena-Glykoproteine | 1 g |
| Gemisch von Cetylstearylalkohol und Natriumcetylstearylosulfuricum und Emulgator (z. B. "Emulgade F") | 17 g |
| Ölsäuredecylester (z. B. "Cetiol V") | 20 g |
| Gemisch von p-Hydroxybenzoesäureestern und Sorbinsäure (z. B. Nipasorbin | 0,5 g |
| H₂O bidest. | 61,5 g |

### Pharmakologische Untersuchungen zur Stimulierung immunologischer Abwehrreaktionen

### Immunmodulierende Aktivität

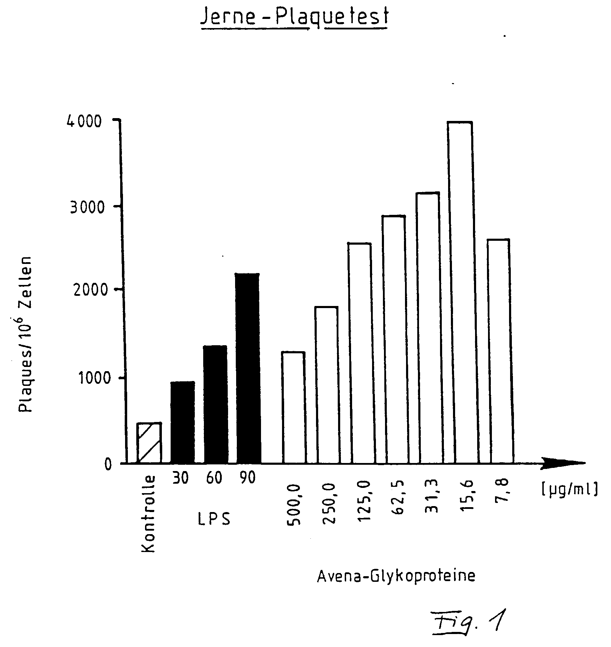

Die immunmodulierende Aktivität wurde anhand der Fähigkeit zur Stimulation der Bildung von Antikörpern durch Lymphozyten der Maus geprüft. In einem - dem Fachmann bekannten - Antikörper-Plaque-Test nach Jerne konnten die Glykoproteine aus Avena eine hochsignifikante Steigerung der Antikörpersynthesen gegen Schaferythrozyten bewirken (Fig. 1). Bei einer Konzentration von 15,6 µg/ml war die Stimulation maximal, etwa um das Achtfache höher als eine unbehandelte Kontrolle. Im Vergleich zu einem bekannten Stimulans, dem Lipopolysaccharid aus Escherichia coli (LPS), war die Stimulation bei vergleichbaren Konzentrationen von 60 bzw. 30 µg/ml doppelt bis dreimal so hoch.

### Mitogene Aktivität

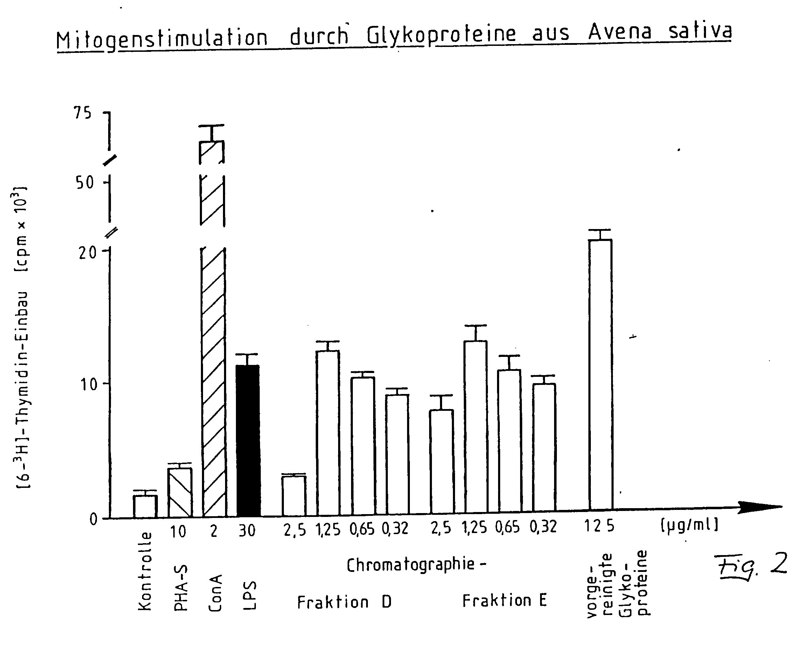

Die mitogene Aktivität von Avena-Glykoproteinen wurde mit Hilfe des Lymphozytentransformationstests an Milzzellen der Maus bestimmt. Gemessen wird hierbei der Einbau von [6-³H]-Thymidin in die Lymphozyten-DNS (Fig. 2). Durch Gelfiltration gewonnene Fraktionen von Glykoproteinen bewirken in einer Konzentration von 1,25 µg/ml eine gleich starke Stimulation wie 30 µg/ml eines bakteriellen B-Zellmitogens (LPS). Mit der gleichen Konzentration an Glykoproteinen werden 50 % der Stimulation von 125 µg/ml vorgereinigten Glykoproteinen erzielt.

### Induktion von Interleukin-1

Das Interleukin-1 ist ein immunologisch unspezifisches Zytokin, das als zentraler Mediator der körpereigenen Abwehr fungiert.

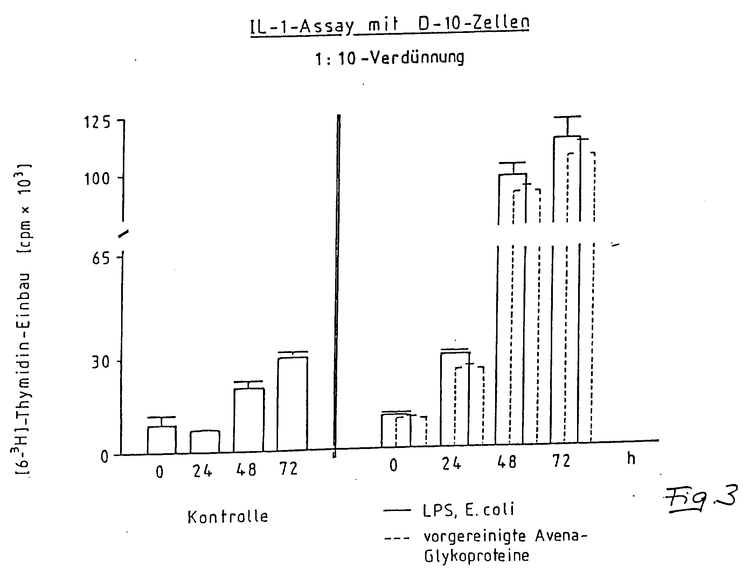

Aufgrund seiner Eigenschaften, Lymphozyten zu aktivieren, die Expression von Akute-Phase-Proteinen zu modulieren und Fieber zu induzieren, besitzt Interleukin-1 eine prominente Bedeutung im Bereich der Entzündung und der Infektion. Monozyten bzw. Makrophagen sind die dominierenden Produzenten von Interleukin-1. Infolgedessen wurde die Prüfung an einer Makrophagenlinie durchgeführt (Fig. 3). Innerhalb eines 72-Stunden-Intervalles bewirken Avena-Glykoproteine eine etwa gleich starke Stimulation wie ein Lipopolysaccharid aus Escherichia coli, das bekanntermaßen Interleukin-1-Ausschüttung induziert. Die Konzentration an vorgereinigten Glykoproteinen betrug 60 µg/ml, für das Lipopolysaccharid 10 µg/ml. Eine unbehandelte Kontrolle erreichte dagegen innerhalb von 72 h nur ein Drittel der Einbaurate.

## Patentansprüche (Patentansprüche für folgende(n) Vertragsstaat(en): BE, CH, DE, FR, GB, IT, LI, LU, NL)

1. Glykoprotein mit immunomodulierenden Eigenschaften und mit einem Molekulargewicht von 38 kD, herstellbar unter nicht-denaturierende Bedingungen aus einem Extrakt der Früchte von Avena sativa, mit einem Anteil von Aminosäureresten von 58,4 % und von Zuckerresten von 41,6 %, mit einem apparenten Molekulargewicht der Polypeptidkette von 22,2 kD, mit neutralen Hexosen in der Zusammensetzung Glukose 45 %, Galaktose 8 %, Mannose 31 %, Arabinose 15 % und einem Gehalt an Osaminen von unter 0,1 % und mit Aminosäureverhältnissen von Aspartat zu Glutamat von 0,97, Alanin zu Valin von 1,03, Valin zu Histidin von 10,6, Valin zu Lysin von 2,3 und Valin zu Arginin von 3,5.

2. Glykoprotein nach Anspruch 1, bei dem die Proteinfraktionen aus etwa 11 Mol-% Asparaginsäure, 11 Mol-% Glutaminsäure und etwa 8 Mol-% Threonin besteht.

3. Glykoprotein nach einem der Ansprüche 1 oder 2, mit einem Verhältnis von Glukose zu Galaktose und Mannose zu Arabinose von etwa 6:1 bzw. 4:2.

4. Verfahren zur Herstellung eines Glykoproteins nach einem der Ansprüche 1 bis 3 aus einem Extrakt der Früchte von Avena sativa, gekennzeichnet durch folgende Verfahrensschritte:
- Ausfilterung von hochmolekularen Anteilen zu deren Weiterverarbeitung;
- Fällen der hochmolekularen Anteile mit einem für Glykoproteine geeigneten Fällmittel;
- Separieren und Lösen des Niederschlags;
- Abtrennen verunreinigender Salze durch Dialyse;
- Trennen der Komponenten nach Molekulargewichtsbereichen und Separieren des Molekulargewichtsbereiches 38 kD.

5. Verfahren nach Anspruch 4, dadurch gekennzeichnet, daß die Ausfilterung der hochmolekularen Anteile durch Diafiltration erfolgt.

6. Verfahren nach einem der Ansprüche 4 oder 5, dadurch gekennzeichnet, daß nach der Dialyse eine Lyophilisation vorgenommen wird.

7. Verfahren nach einem der Ansprüche 4 bis 6, dadurch gekennzeichnet, daß das Trennen der Komponenten nach Molekulargewichtsbereichen durch eine Chromatographie an Gelen erfolgt.

8. Verfahren nach Anspruch 7, dadurch gekennzeichnet, daß die der Chromatographie zugeführte Lösung gepuffert ist und daß die Elution mit dem gleichen Puffer durchgeführt wird.

9. Verfahren nach Anspruch 8, dadurch gekennzeichnet, daß ein 0,1 M Ammoniumcarbonat-Puffer verwendet wird.

10. Verfahren nach einem der Ansprüche 4 bis 9, dadurch gekennzeichnet, daß vor der Selektion der hochmolekularen Bestandteile Lipide und Nukleinsäuren gefällt und abgetrennt werden.

11. Verfahren nach Anspruch 10, dadurch gekennzeichnet, daß zur Fällung quaternäres Ammonium verwendet wird.

12. Arzneimittel enthaltend das Glykoprotein nach einem der Ansprüche 1 bis 3 als Wirkstoff.

13. Verwendung des Glykoproteins nach einem der Ansprüche 1 bis 3 zur Herstellung eines immunmodulierenden Arzneimittels.

## Patentansprüche (Patentansprüche für folgende(n) Vertragsstaat(en): ES)

1. Verfahren zur Herstellung eines Glykoproteins unter nicht-denaturierenden Bedingungen mit immunomodulierenden Eigenschaften und mit einem Molekulargewicht von 38 kD, mit einem Anteil von Aminosäureresten von 58,4 % und von Zuckerresten von 41,6 %, mit einem apparenten Molekulargewicht der Polypeptidkette von 22,2 kD, mit neutralen Hexosen in der Zusammensetzung Glucose 45 %, Galactose 8 %, Mannose 31 %, Arabinose 15 % und einem Gehalt an Osaminen von unter 0,1 % und mit Aminosäureverhältnissen von Aspartat zu Glutamat von 0,97, Adanin zu Valin von 1,03, Valin zu Histidin von 10,6, Valin zu Lysin von 2,3 und Valin zu Arginin von 3,5, aus einem Extrakt von Avena sativa mit folgenden Verfahrensschritten:
- Ausfilterung von hochmolekularen Anteilen zu deren Weiterverarbeitung;
- Fällen der hochmolekularen Anteile mit einem für Glykoproteine geeigneten Fällmittel;
- Separieren und Lösen des Niederschlags;
- Abtrennen verunreinigender Salze durch Dialyse;
- Trennen der Komponenten nach Molekulargewichtsbereichen und Separieren des Molekulargewichtsbereichs 38 kD.

2. Verfahren nach Anspruch 1, dadurch gekennzeichnet, daß die Ausfilterung der hochmolekularen Anteile durch Diafiltration erfolgt.

3. Verfahren nach Anspruch 1 oder 2, dadurch gekennzeichnet, daß nach der Dialyse eine Lyophilisation vorgenommen wird.

4. Verfahren nach einem der Ansprüche 1 bis 3, dadurch gekennzeichnet, daß das Trennen der Komponenten nach Molekulargewichtsbereichen durch eine Chromatographie an Gelen erfolgt.

5. Verfahren nach Anspruch 4, dadurch gekennzeichnet, daß die der Chromatographie zugeführte Lösung gepuffert ist und daß die Elution mit dem gleichen Puffer durchgeführt wird.

6. Verfahren nach Anspruch 5, dadurch gekennzeichnet, daß ein 0,1 M Ammoniumcarbonat-Puffer verwendet wird.

7. Verfahren nach einem der Ansprüche 1 bis 6, dadurch gekennzeichnet, daß vor der Selektion der hochmolekularen Bestandteile Lipide und Nukleinsäuren gefällt und abgetrennt werden.

8. Verfahren nach Anspruch 7, dadurch gekennzeichnet, daß zur Fällung quaternäres Ammonium verwendet wird.

## Claims (Claims for the following Contracting State(s): BE, CH, DE, FR, GB, IT, LI, LU, NL)

1. Glycoprotein having immune-modulating properties and having a molecular weight of 38 kD, obtainable under non-denaturing conditions from an extract of the fruits of Avena sativa, having a 58.4% amino acid radicals content and a 41.6% sugar radicals content, having a polypeptide chain apparent molecular weight of 22.2 kD, having neutral hexoses in the composition glucose 45%, galactose 8%, mannose 31%, arabinose 15% and an osamines content of less than 0.1% and having amino acid ratios of aspartate to glutamate of 0.97, alanine to valine of 1.03, valine to histidine of 10.6, valine to lysine of 2.3 and valine to arginine of 3.5.

2. Glycoprotein according to Claim 1, in which the protein fractions comprise approximately 11 mol-% aspartic acid, 11 mol-% glutamic acid and approximately 8 mol-% threonine.

3. Glycoprotein according to one of Claims 1 or 2, having a ratio of glucose to galactose and of mannose to arabinose of approximately 6 : 1 and 4 : 2, respectively.

4. Process for obtaining a glycoprotein according to one of Claims 1 to 3 from an extract of the fruits of Avena sativa, characterised by the following process steps:
- filtering out high molecular weight constituents in order to process them further;
- precipitating the high molecular weight constituents with a precipitant suitable for glycoproteins;
- isolating and dissolving the precipitate;
- removing salt impurities by dialysis;
- separating the components by molecular weight regions and isolating the 38 kD molecular weight region.

5. Process according to Claim 4, characterised in that the high molecular weight constituents are filtered out by diafiltration.

6. Process according to one of Claims 4 or 5, characterised in that following the dialysis a lyophilisation is performed.

7. Process according to one of Claims 4 to 6, characterised in that the components are separated according to molecular weight regions by gel chromatography.

8. Process according to Claim 7, characterised in that the solution which is supplied to chromatography is buffered and that elution is performed with the same buffer.

9. Process according to Claim 8, characterised in that a 0.1 M ammonium carbonate buffer is used.

10. Process according to one of Claims 4 to 9, characterised in that lipids and nucleic acids are precipitated and removed before the high molecular components are selected.

11. Process according to Claim 10, characterised in that quaternary ammonium is used for the precipitation.

12. Medicaments containing as an active ingredient the glycoprotein according to one of Claims 1 to 3.

13. Use of the glycoprotein according to one of Claims 1 to 3 to obtain an immuno-modulating medicament.

## Claims (Claims for the following Contracting State(s): ES)

1. Process for obtaining under non-denaturing conditions from an extract of Avena sativa a glycoprotein having immuno-modulating properties and having a molecular weight of 38 kD, having a 58.4% amino acid radicals content and a 41.6% sugar radicals content, having a polypeptide chain apparent molecular weight of 22.2 kD, having neutral hexoses in the composition glucose 45%, galactose 8%, mannose 31%, arabinose 15% and an osamines content of less than 0.1% and having amino acid ratios of aspartate to glutamate of 0.97, alanine to valine of 1.03, valine to histidine of 10.6, valine to lysine of 2.3 and valine to arginine of 3.5, having the following process steps:
- filtering out high molecular weight constituents in order to process them further;
- precipitating the high molecular weight constituents with a precipitant suitable for glycoproteins;
- isolating and dissolving the precipitate;
- removing salt impurities by dialysis;
- separating the components by molecular weight regions and isolating the 38 kD molecular weight region.

2. Process according to claim 1, characterised in that the high molecular weight constituents are filtered out by diafiltration.

3. Process according to Claim 1 or 2, characterised in that following the dialysis a lyophilisation is performed.

4. Process according to one of Claims 1 to 3, characterised in that the components are separated according to molecular weight regions by gel chromatography.

5. Process according to Claim 4, characterised in that the solution which is supplied to chromatography is buffered and that elution is performed with the same buffer.

6. Process according to Claim 5, characterised in that a 0.1 M ammonium carbonate buffer is used.

7. Process according to one of Claims 1 to 6, characterised in that lipids and nucleic acids are precipitated and removed before the high molecular weight components are selected.

8. Process according to Claim 7, characterised in that quaternary ammonium is used for the precipitation.

## Revendications (Revendications pour l'(les) Etat(s) contractant(s) suivant(s): BE, CH, DE, FR, GB, IT, LI, LU, NL)

1. Glycoprotéine présentant des propriétés immunomodulatrices et un poids moléculaire de 38 kD, pouvant être préparée dans des conditions non dénaturantes à partir d'un extrait des fruits d'Avena sativa, contenant une proportion de 58,4 % de restes d'acides aminés et 41,6 % de restes de sucres, avec un poids moléculaire apparent de la chaîne polypeptidique de 22,2 kD, contenant des hexoses neutres composés à 45 % de glucose, 8 % de galactose, 31 % de mannose, 15 % d'arabinose, avec une teneur en osamines inférieure à 0,1 % et des rapports des acides aminés aspartate sur glutamate de 0,97, alanine sur valine de 1,03, valine sur histidine de 10,6, valine sur lysine de 2,3 et valine sur arginine de 3,5.

2. Glycoprotéine selon la revendication 1, dans laquelle les fractions protéiniques se composent d'environ 11 % en moles d'acide aspartique, de 11 % en moles d'acide glutamique et d'environ 8 % en moles de thréonine.

3. Glycoprotéine selon la revendication 1 ou 2, avec un rapport glucose sur galactose et mannose sur arabinose d'environ 6:1 et de préférence 4:2.

4. Procédé de préparation d'une glycoprotéine selon l'une quelconque des revendications 1 à 3 à partir d'un extrait de fruits d'Avena sativa, caractérisé par les étapes de procédé suivantes :
à Extraction par filtration des portions de haut poids moléculaire afin d'en poursuivre le traitement;
à Précipitation des fractions de haut poids moléculaire au moyen d'un agent précipitant approprié pour les glycoprotéines;
à Isolation et dissolution du précipité;
à Elimination par dialyse des sels constituant des impuretés;
à Séparation des composants par classes de poids moléculaire et isolation de la classe de poids moléculaire 38 kD.

5. Procédé selon la revendication 4, caractérisé en ce que l'extraction par filtration des fractions de haut poids moléculaire se fait par filtration dialytique.

6. Procédé selon l'une des revendicationq 4 ou 5, caractérisé en ce que la dialyse est suivie d'une lyophilisation.

7. Procédé selon l'une quelconque des revendications 4 à 6, caractérisé en ce que la séparation des composants par classes de poids moléculaire se fait par chromatographie sur des gels.

8. Procédé selon la revendication 7, caractérisé en ce que la solution soumise à la chromatographie est tamponnée et en ce que l'élution est réalisée avec le même tampon.

9. Procédé selon la revendication 8, caractérisé en ce que le tampon utilisé est du carbonate d'ammonium 0,1 M.

10. Procédé selon l'une quelconque des revendications 4 à 9, caractérisé en ce que des lipides et des acides nucléiques sont précipités et séparés avant la sélection des composants de haut poids moléculaire.

11. Procédé selon la revendication 10, caractérisé en ce qu'un ammonium quaternaire est utilisé pour la précipitation.

12. Médicament contenant comme principe actif la glycoprotéine selon l'une quelconque des revendications 1 à 3.

13. Utilisation de la glycoprotéine selon l'une quelconque des revendications 1 à 3 pour préparer un médicament immunomodulateur.

## Revendications (Revendications pour l'(les) Etat(s) contractant(s) suivant(s): ES)

1. Procédé de préparation dans des conditions non dénaturantes d'une glycoprotéine présentant des propriétés immunomodulatrices et un poids moléculaire de 38 kD, contenant une proportion de 58,4 % de restes d'acides aminés et 41,6 % de restes de sucres, avec un poids moléculaire apparent de la chaîne polypeptidique de 22,2 kD, contenant des hexoses neutres composés à 45 % de glucose, 8 % de galactose, 31 % de mannose, 15 % d'arabinose, avec une teneur en osamines inférieure à 0,1 % et des rapports des acides aminés aspartate sur glutamate de 0,97, alanine sur valine de 1,03, valine sur histidine de 10,6, valine sur lysine de 2,3 et valine sur arginine de 3,5, à partir d'un extrait des fruits d'Avena sativa, avec les étapes de procédé suivantes :
à Extraction par filtration des portions de haut poids moléculaire afin d'en poursuivre le traitement;
à Précipitation des fractions de haut poids moléculaire au moyen d'un agent précipitant approprié pour les glycoprotéines;
à Isolation et dissolution du précipité;
à Elimination par dialyse des sels constituant des impuretés;
à Séparation des composants par classes de poids moléculaire et isolation de la classe de poids moléculaire 38 kD.

2. Procédé selon la revendication 1, caractérisé en ce que l'extraction par filtration des fractions de haut poids moléculaire se fait par filtration dialytique.

3. Procédé selon la revendication 1 ou 2, caractérisé en ce que la dialyse est suivie d'une lyophilisation.

4. Procédé selon l'une quelconque des revendications 1 à 3, caractérisé en ce que la séparation des composants par classes de poids moléculaire se fait par chromatographie sur des gels.

5. Procédé selon la revendication 4, caractérisé en ce que la solution soumise à la chromatographie est tamponnée et en ce que l'élution est réalisée avec le même tampon.

6. Procédé selon la revendication 5, caractérisé en ce que le tampon utilisé est du carbonate d'ammonium 0,1 M.

7. Procédé selon l'une quelconque des revendications 1 à 6, caractérisé en ce que des lipides et des acides nucléiques sont précipités et séparés avant la sélection des composants de haut poids moléculaire.

8. Procédé selon la revendication 7, caractérisé en ce qu'un ammonium quaternaire est utilisé pour la précipitation.
